Europäisches Patentamt

European Patent Office

Office européen des brevets

(19)

(11)  **EP 1 183 225 B1**

(12)  **EUROPÄISCHE PATENTSCHRIFT**

(45) Veröffentlichungstag und Bekanntmachung des Hinweises auf die Patenterteilung:
**03.09.2003   Patentblatt 2003/36**

(51) Int Cl.$^7$: **C07C 67/03**, C07C 69/52

(21) Anmeldenummer: 00943778.1

(86) Internationale Anmeldenummer:
**PCT/EP00/05255**

(22) Anmeldetag: **07.06.2000**

(87) Internationale Veröffentlichungsnummer:
**WO 00/075098 (14.12.2000 Gazette 2000/50)**

(54) **VERFAHREN ZUR HERSTELLUNG VON FETTSÄUREESTERN EINWERTIGER ALKYLALKOHOLE**

METHOD FOR PRODUCING FATTY ACID ESTERS OF MONOVALENT ALKYL ALCOHOLS

PROCEDE DE PRODUCTION D'ESTERS D'ACIDES GRAS D'ALCOOLS ALKYLIQUES MONOVALENTS

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **07.06.1999   DE 19925871**

(43) Veröffentlichungstag der Anmeldung:
**06.03.2002   Patentblatt 2002/10**

(73) Patentinhaber: **AT Agrar-Technik GmbH**
**72667 Schlaitdorf (DE)**

(72) Erfinder: **TÜRCK, Ralf**
**D-97320 Mainstockheim (DE)**

(74) Vertreter: **HOFFMANN - EITLE**
**Patent- und Rechtsanwälte**
**Arabellastrasse 4**
**81925 München (DE)**

(56) Entgegenhaltungen:
**EP-A- 0 131 991          EP-A- 0 523 767**
**DE-C- 4 436 517**

## Beschreibung

Fettsäureester einwertiger Alkylalkohole finden in der chemischen und pharmazeutischen Industrie sowohl als Rohstoff als auch als Zwischenprodukte vielfältige Verwendung. Darüber hinaus kommen derartige Verbindungen auch in der Nahrungsmittelindustrie und seit kurzer Zeit insbesondere auch als Dieselkraftstoff zum Einsatz.

Zur Herstellung dieser Verbindungen sind verschiedene Wege ausgehend von Erdöl oder nachwachsenden Rohstoffen vorgeschlagen worden. In der Praxis kommt der Darstellung solcher Ester aus pflanzlichen oder tierischen Ölen und Fetten nicht zuletzt aus Gründen des Umweltschutzes besondere Bedeutung zu.

Die bisherigen Verfahren basieren in der Regel auf der bereits seit längerem bekannten basenkatalysierten Umesterung von Fettsäureestern mehrwertiger Alkohole, insbesondere von Fettsäureglyceriden (siehe z.B. J. Am. Oil Chem. Soc. 61 (1984), 343 oder Ullmann, Enzyklopädie der Technischen Chemie, 4. Auflage, Band 11, Seite 432).

Die in den letzten Jahren vorgeschlagenen technischen Verfahren zur Herstellung von Fettsäureestern einwertiger Alkylalkohole durch basenkatalysierte Umesterung von Fettsäureestern mehrwertiger Alkohole, wie z.B. in DE-3932514 oder DE-4209779 offenbart, betreiben zum Teil einen hohen apparativen Aufwand und/oder arbeiten unter kostenintensiven Bedingungen, d.h. bei hoher Temperatur und/oder hohem Druck und schließen auch oftmals aufwendige Aufarbeitungsschritte, wie Destillationen, ein. Aus diesen Gründen lassen sich derartige Verfahren wirtschaftlich nur im großtechnischen Maßstab ab etwa 50.000 Jahrestonnen und mehr durchführen. Für kleine Anlagen von etwa 500-5.000 Jahrestonnen eignen sich diese Verfahren nicht.

Speziell auf die Möglichkeiten von solchen Kleinanlagen abgestimmte Verfahren sind z.B. aus AT-386222, AT-397966, AT-387399, DE-3727981, DE-3020612, DE-3107318 oder WO 92/00268 bekannt. All diese Verfahren basieren auf der erwähnten Umesterungsreaktion und streben eine möglichst weitgehende Vereinfachung an, um Kosten zu senken und so auch in kleinem Maßstab wirtschaftlich arbeiten zu können. Insbesondere versuchen diese Verfahren auf energieintensive und apparativ aufwendige Trennungsschritte, wie Destillationen, zu verzichten. Als Mittel der Wahl hat sich für die Abtrennung des Produkts oder von Zwischenprodukten hier die Phasentrennung etabliert.

Bei der Verwendung von unraffinierten Ausgangsölen, wie etwa nativen Ölen, ergeben sich bei diesen Verfahren jedoch zwei wichtige Probleme. Native Öle, insbesondere pflanzlichen Ursprungs, enthalten in der Regel Schleimstoffe wie etwa Phosphatide. Diese Stoffe sind oberflächenaktiv und werden deshalb zum Teil in der Nahrungsmittelindustrie als Emulgatoren verwendet. Im Zusammenhang mit den hier angesprochenen Verfahren zur Herstellung von Fettsäureestern ergibt sich aus dieser Eigenschaft das Problem, daß diese Verbindungen die Phasentrennung nachteilig beeinflussen. Dementsprechend müssen für einen reibungslosen Verfahrensablauf bereits entschleimte Ausgangsöle eingesetzt werden, oder aber zusätzliche Verfahrensschritte durchgeführt werden, die in der Regel auch zusätzliche Anlagenteile erfordern, um die durch die unvollständige oder langsame Phasentrennung verursachten Ausbeuteverluste zu vermindern. Höhere Schleimstoff- und insbesondere Phosphatidgehalte im Ausgangsöl bedingen daher in herkömmlichen Verfahren höhere Kosten.

Des weiteren enthalten die oben genannten Öle oftmals auch nicht unerhebliche Mengen an freien Fettsäuren, deren Gegenwart sich ebenfalls negativ auswirkt. Als freie Säuren reagieren diese Stoffe nämlich unter Seifenbildung mit dem für die Umesterung zugegebenen basischen Katalysator. Hierdurch wird ein Teil des Katalysators neutralisiert und steht somit nicht mehr für die Umesterungsreaktion zur Verfügung. Um dieses Problem zu lösen, können die freien Fettsäuren vor der eigentlichen Umesterung neutralisiert und/oder entfernt werden, oder aber es muß eine entsprechend größere Menge an basischem Katalysator zugegeben werden (WO 92/00268).

Dieses Vorgehen hat jedoch zur Folge, daß wegen des erhöhten Katalysatorbedarfs zusätzliche Kosten entstehen und daß dadurch im Reaktionsgemisch nicht unerhebliche Mengen an Seifen gebildet werden. Da auch diese Verbindungen oberflächenaktive Eigenschaften besitzen, erschweren sie die Phasentrennung und müssen abgetrennt werden, wodurch wiederum zusätzliche Kosten entstehen.

EP-A-0 131 991 offenbart ein Verfahren zur Umesterung von Fettsäureestern, welches unter Basenkatalyse durchgeführt wird. Weiterhin werden bei diesem Verfahren die eingesetzten Fettsäureester durch Vorbehandlung mit Base von schädlichen Begleitstoffen befreit. Die Umesterungsreaktion wird in einem Gegenstromreaktor durchgeführt, und ist somit für kleine Anlagen nicht praktikabel.

Der vorliegenden Erfindung liegt daher die Aufgabe zugrunde, ein möglichst einfaches und wirtschaftliches Verfahren zur Herstellung von Fettsäureestern einwertiger Alkylalkohole bereitzustellen, das möglichst geringe Anforderungen an die Ausgangsöle stellt, und gleichzeitig hohe Ausbeuten garantiert.

Diese Aufgabe wird durch ein Verfahren zur Herstellung von Fettsäureestern einwertiger Alkylalkohole durch basenkatalysierte Umesterung von Triglyceriden aus natürlichen oder synthetischen Ölen und/oder Fetten, die als störende Begleitstoffe freie Fettsäuren und Phosphatide enthalten, gelöst,

wobei man zunächst die Öle und/oder Fette mit einer nicht-mischbaren basischen Glycerin-Phase behandelt, so daß die freien Fettsäuren neutralisiert werden und in die Glycerin-Phase übergehen, und dann

die Triglyceride nach Abtrennung von der Glycerin-Phase mit einwertigen Alkoholen unter Verwendung einer Base als Katalysator in einem Rührkessel zu den Fettsäureestern umestert,

dadurch gekennzeichnet, daß

man die bei der Umesterung der Triglyceride anfallende basische Glycerin-Phase nach Abtrennung der Fettsäureester für die Behandlung der Öle und/oder Fette zur Entfernung der freien Fettsäuren verwendet, wobei die Mindestmenge an eingesetztem Katalysator bezogen auf 1000 g des zu behandelnden Öls in Abhängigkeit von der Säurezahl und der mittleren Molmasse des Öls gemäß Gleichungen (I) - (III) berechnet wird:

bei einer Säurezahl, die Ungleichung (I) erfüllt

$$SZ < (0{,}084 \text{ mol}/1000 \text{ g Öl}) * M(KOH) * Y \qquad (I)$$

gemäß Gleichung (II)

$$\text{Mindestmenge Kat}/1000 \text{ g Öl} = 0{,}088 \text{ mol}/1000 \text{ g Öl} * Y \qquad (II)$$

andernfalls gemäß Gleichung (III)

$$\text{Mindestmenge Kat}/1000 \text{ g Öl} =$$

$$(SZ / M(KOH)) * (0{,}088 / 0{,}084) \qquad (III)$$

mit

Y = (880 g/mol) / (mittlere Molmasse des eingesetzten Öls) und
SZ = Säurezahl des eingesetzten Öls [(g KOH)/(1000 g Öl)].

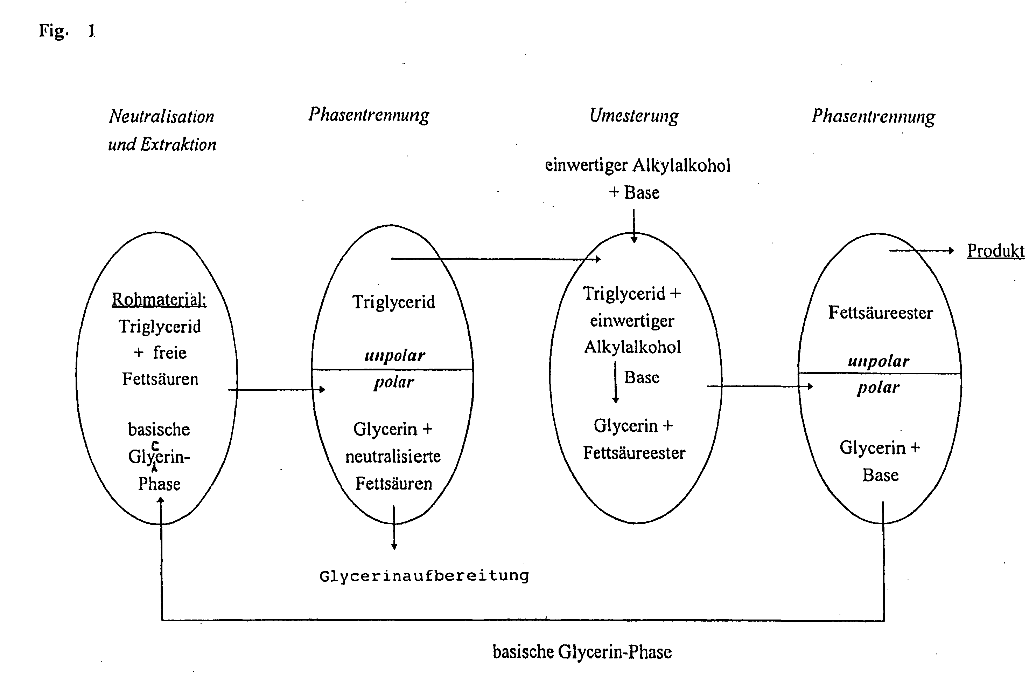

Das erfindungsgemäße Verfahren wird durch Fig. 1 näher erläutert. Die erzielten Vorteile liegen insbesondere darin, daß Ausgangsöle mit einem hohen Gehalt an freien Fettsäuren, die zusätzlich auch Schleimstoffe enthalten können, problemlos zu Fettsäureestern einwertiger Alkylalkohole verarbeitet werden können. Weiterhin werden im erfindungsgemäßen Verfahren nur katalytische Mengen Base benötigt, so daß zusätzliche Aufwendungen für Katalysatormaterial zur Neutralisation der freien Säuren in der Regel entfallen, da die im Umesterungsschritt anfallende Katalysatormenge im Extraktionsschritt für den Neutralisationsschritt zur Verfügung steht. Trotz dieser Vereinfachungen liefert das erfindungsgemäße Verfahren ein Produkt mit hoher Reinheit und in sehr guter Ausbeute. Der Einsatz des im Umesterungsschritt anfallenden und als polare und schwerere Phase abtrennbaren basischen Glycerin-Phase, welche im wesentlichen Glycerin und basischen Katalysator enthält, im Neutralisationsschritt hat insbesondere den Vorteil, daß keine zusätzlichen Verunreinigungen mit einer neuen Base im Neutralisationsschritt eingeschleppt werden.

In dem erfindungsgemäßen Verfahren lassen sich als Ausgangsmaterial Triglyceride beliebiger Herkunft verarbeiten. Beispielhaft seien hier tierische oder pflanzliche Fette und Öle genannt, wie sie herkömmlicherweise zur Herstellung von Fettsäureestern verwendet werden. Ein besonders bevorzugtes Ausgangsmaterial ist Rapsöl.

Überraschenderweise wurde gefunden, daß sich das aus der Umesterung stammende basische Glycerin-Phase hervorragend dazu eignet, in einem Vorbehandlungsschritt die freien Fettsäuren aus dem Ausgangsstoff zu extrahieren. Die dabei wirkende Chemikalie ist der alkalische Umesterungskatalysator, der in diesem Reaktionsschritt die freien, unpolaren Fettsäuren in Form von ionischen Seifen im polaren Glycerin auflöst. Insofern wird der Katalysator zuerst in der Umesterung und anschließend in der Neutralisation und Extraktion der freien Fettsäuren eingesetzt.

Die erfindungsgemäß erzielten Vorteile liegen insbesondere darin, daß Ausgangsöle mit einem hohen Gehalt an freien Fettsäuren problemlos und mit geringsten Ausbeuteverlusten zu Fettsäureestern einwertiger Alkylalkohole verarbeitet werden können. Weiterhin ist eine bei anderen Verfahren notwendige vorherige Entschleimung der Pflanzenöle nicht notwendig.

Im ersten Verfahrensschritt wird erfindungsgemäß das Ausgangsmaterial mit basischer Glycerin-Phase verrührt. Dabei werden die freien Fettsäuren und Restwasser weitgehend extrahiert.

Für diese Vorbehandlung wird das Ausgangsmaterial mit der basischen Glycerin-Phase 1 - 60 min, bevorzugt 5 - 15 min, gemischt, wozu vorzugsweise ein Rührkessel eingesetzt wird.

Die Reaktionsbedingungen dieser Vorbehandlung sind lediglich durch den Erstarrungspunkt des Rohstoffes beschränkt. Unter dem Gesichtspunkt der Wirtschaftlichkeit sollte dies bei Umgebungstemperatur und Umgebungsdruck, bei höheren Erstarrungspunkten etwa 10 °C oberhalb dieses Punktes und Umgebungsdruck, erfolgen.

Die Wahl der basischen Glycerin-Phase ist variabel. Es muß lediglich gewährleistet sein, das noch mindestens soviel freier Katalysator vorliegt, daß alle freien Fettsäuren neutralisiert werden können. In der Regel reicht der Anteil an freiem Katalysator von der aus der Umesterung kommenden basischen Glycerin-Phase auch für Rohstoffe mit hohem Anteil an freien Fettsäuren aus. Die basische Glycerin-Phase hat einen Glyceringehalt von 20 - 99%, bevorzugt 40 - 60 %. Der Katalysatorgehalt liegt zwischen 1 - 30 %, bevorzugt 5 - 10 %. Der Anteil an Umesterungsalkohol liegt zwischen 5 - 40 %, bevorzugt 15 - 25 %. Die Zugabemenge an basischer Glycerin-Phase bezogen auf den Rohstoff schwankt zwischen 1 - 100 %, bevorzugt 5 - 15 %. Die basische Glycerin-Phase kann bei Bedarf auch durch Zugabe von festem alkalischem Katalysator oder eines Alkohol/ Katalysatorgemisches ergänzt werden. Das Glycerinextraktionsmittel kann auch durch direktes Mischen von Glycerinen verschiedener Qualitäten, technisch bis Pharma, und alkalischem Katalysator mit oder ohne Alkohole hergestellt werden. Als alkalischer Katalysator kommen alle für die Umesterungsreaktionen eingesetzten Katalysatoren in Frage, Metallhydroxide und/oder -alkoholate insbesondere von Metallen aus der I. bis III. Hauptgruppe des Periodensystems, bevorzugt NaOH, KOH und Natriumalkoholate wie z.B. Natriumethylat.

Nach der vorstehend beschriebenen Extraktionsstufe kann das Reaktionsgemisch durch einfache Sedimentation getrennt werden, und anschließend sofort der Umesterung zugeführt werden. Übliche Sedimentationszeiten sind 15 min - 72 h, bevorzugt 1 - 3h. Die Temperatur des Rohstoffes sollte oberhalb des Erstarrungspunktes liegen. Bei Pflanzenölen wie Raps-, Soja- und Sonnenblumenöl ist eine Umgebungstemperatur von 20 °C ausreichend, bei Tierkörper- und Friteusenfetten sind 40 - 50 °C realistisch. Allgemein sollte die Sedimentationstemperatur zwischen 0 - 100 °C, bevorzugt 20 - 40 °C liegen.

Um die Rohstoffverwertung zu optimieren bietet es sich überdies an, die in der basischen Glycerin-Phase enthaltenen freien Fettsäuren bzw. deren Salze gemäß WO 95/02661 abzutrennen und mit Alkylalkohol zu verestern. Dies geschieht bevorzugt unter saurer Katalyse. Das bei dieser sauren Nachveresterung erhaltene Reaktionsgemisch kann dem in den folgenden Abschnitten beschriebenen Umesterungsgemisch gegen Ende der Umesterungsreaktion beigemischt werden. Hierbei ist jedoch zu beachten, daß der saure Katalysator der Nachveresterung der freien Fettsäuren einen Teil des für die Umesterung benötigten basischen Katalysators neutralisiert.

Nach Abschluß der Vorbehandlung kann das erhaltene Triglycerid direkt mit Base zur Durchführung der Umesterung der Fettsäureester mehrwertiger Alkohole zu Fettsäureestern einwertiger Alkylalkohole versetzt werden. Zu diesem Zweck wird es in einen Rührkessel überführt.

Die Menge an Alkylalkohol, die für die Umesterung eingesetzt wird, verteilt sich auf 1 bis 10, bevorzugt 2 Umesterungsstufen. Insgesamt werden für das erfindungsgemäße Verfahren etwa 1,05 - 2 mol Alkohol pro mol gebundener Fettsäureanteile eingesetzt, bevorzugt 1,2 - 1,4 mol. Bei der üblichen zweistufigen Verfahrensausführung werden in der ersten Stufe 40 - 99%, bevorzugt 90 - 95 %, und in der zweiten Stufe 1 - 60%, bevorzugt 5 - 10 % eingesetzt.

Die Art des einzusetzenden Alkylalkohols ist im vorliegenden Verfahren nicht weiter beschränkt. Bevorzugt sind jedoch lineare, verzweigte oder cyclische Alkylalkohole mit 1 - 10 Kohlenstoffatomen, besonders bevorzugt ist Methanol.

Die die Umesterungsreaktion katalysierende Base, bei der es sich vorteilhaft um Metallhydroxide und/oder -alkoholate insbesondere von Metallen aus der I. bis III. Hauptgruppe des Periodensystems handelt, kann dem Reaktionsgemisch sowohl in fester Form als auch in Form einer alkoholischen Lösung zugesetzt werden. Kaliumhydroxid ist als Base besonders bevorzugt.

Falls eine alkoholische Lösung verwendet wird, sind solche bevorzugt, die 25 - 50 Gew.-% Base, bezogen auf das Gesamtgewicht der Lösung, enthalten. Die auf diesem Wege eingetragene Alkoholmenge ist natürlich in die Gesamtalkoholmenge mit einzubeziehen.

Die Gesamtmenge an Base, die in diesem Verfahrensschritt zugegeben wird, hängt wiederum von der weiteren Verfahrensführung und der Beschaffenheit des Ausgangsmaterials ab. Bei einstufiger Umesterung wird in dieser Stufe natürlich die Gesamtmenge an basischem Umesterungskatalysator eingesetzt, wohingegen bei einer zweistufigen Umesterung in dieser Stufe nur 20 - 95%, und bevorzugt 80 - 90%, der Gesamtmenge verwendet werden.

Die Mindestmenge an basischem Umesterungskatalysator ist bei niedrigem Gehalt an freien Fettsäuren (niedrige Säurezahl; Ungleichung (I) ist erfüllt) durch die Umesterungsreaktion vorgegeben und kann gemäß Gleichung (II) bestimmt werden. Bei höheren Säurezahlen (Ungleichung (I) ist nicht erfüllt) wird die Neutralisation der freien Fettsäuren zum bestimmenden Schritt für die Berechnung der erforderlichen Katalysatormenge. In diesem Fall sollte die einzusetzende Mindestmenge an Base anhand von Gleichung (III) ermittelt werden. Der Zahlenwert 0,088 mol/1000 g Öl ist ein empirisch ermittelter Mindestwert, der so bestimmt wurde, daß eine akzeptable Reaktionsgeschwindigkeit gewährleistet wird. Von dieser Menge stehen 0,084 mol/1000 g Öl für die Neutralisation der freien Fettsäuren zu Verfügung; das Verhältnis 0,084/0,088 kann somit als Rezyklierungsausbeute verstanden werden.

Nach ökonomischen Aspekten betrachtet ist es wenig sinnvoll, daß 10-fache der theoretisch eizusetzenden Mindestmenge zu überschreiten. Als besonders vorteilhaft hat sich die Verwendung des 1 bis 4-fachen der theoretisch einzusetzenden Mindestmenge an basischem Umesterungskatalysator herausgestellt.

Die Reaktionszeit in dieser Stufe beträgt 5-60 min, und bevorzugt 25-35 min. Da auch diese Stufe vorzugsweise bei Umgebungstemperatur und -druck durchgeführt wird, ist die genaue Reaktionsdauer an die jeweiligen Bedingungen anzupassen.

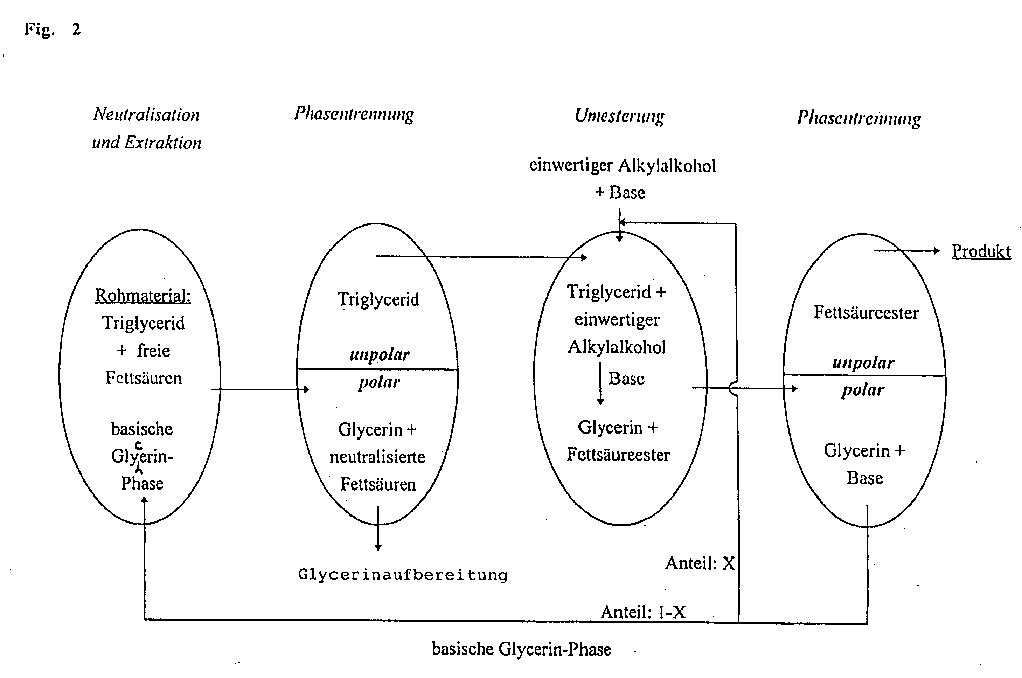

Insbesondere bei niedrigen Säurezahlen ist eine weitere Reduktion des Katalysatorbedarfs möglich indem ein Anteil X der bei der Umesterung anfallenden basischen Glycerin-Phase nach Abtrennung der Fettsäureester für die Umesterungsreaktion wiederverwendet wird. Diese Verfahrensführung ist in Fig. 2 schematisch dargestellt.

Eine derartige Wiederverwendung der basischen Glycerin-Phase ist lediglich bei einer Säurezahl sinnvoll, die Ungleichung (IV) erfüllt:

$$SZ < (0,084 \text{ mol/1000 g Öl}) * M(KOH) * Y \qquad (IV)$$

Die einzusetzende Mindestmenge an Katalysator (bezogen auf 1000 g des zu behandelnden Öls) sowie der Anteil X der für die Umesterung wiedereinzusetzenden basischen Glycerin-Phase müssen in Abhängigkeit von der Säurezahl und der mittleren Molmasse des Öls so gewählt werden, daß Gleichungen (V) und (VI) gleichzeitig erfüllt werden:

$$\text{Mindestmenge Kat/1000 g Öl} =$$

$$(0,088 \text{ mol/1000 g Öl} - (X * 0,084 \text{ mol/1000 g Öl})) * Y \qquad (V)$$

$$\text{Mindestmenge Kat/1000 g Öl} =$$

$$(SZ / M(KOH)) * (0,088 / (0,084 * (1 - X))) \qquad (VI)$$

mit

$Y$ = (880 g/mol) / (mittlere Molmasse des eingesetzten Öls)
$SZ$ = Säurezahl des eingesetzten Öls [(g KOH)/(1000 g Öl)]
$X$ = Anteil der basischen Glycerin-Phase, der für die Umesterung wiederverwendet wird.

Gleichung (V) entspricht Gleichung (II), der zweite Term berücksichtigt die wiederverwertete Menge an basischer Glycerin-Phase im Umesterungsschritt. Gleichung (VI) ist das Pendant zu Gleichung (III). Die erforderliche Mindestmenge nach dieser Gleichung ist höher als die nach Gleichung (III), da lediglich der Anteil 1-X der basischen Glycerin-Phase für die Neutralisation der freien Fettsäuren eingesetzt wird. Die Wiederverwendung der basischen Glycerin-Phase für die Umesterung findet ihre Grenzen dort wo nicht mehr ausreichend Base für die Neutralisation der freien Fettsäuren im Anteil 1-X vorhanden ist. Daher muß die optimale Kombination von eingesetzter Menge an Base und Wiederverwendungsanteil X unter gleichzeitiger Berücksichtigung von Gleich (V) und (IV) bestimmt werden.

Durch Gleichsetzen von Gleichungen (V) und (VI) und Umformen kann eine quadratische Gleichung erhalten werden, deren einzige physikalisch sinnvolle Lösung einen Wert von X im Bereich von 0 bis 1 liefert. Dieser, maximal für die Umesterung einzusetzende mögliche Anteil X kann im folgenden dann in Gleichungen (V) oder (VI) eingesetzt werden um die erforderliche Mindestmenge an Katalysator zu bestimmen.

Setzt man für:

$SZ / (M(KOH) * Y) = a$
$0.088 \text{ mol/1000 g} = b$
$0.084 \text{ mol/1000 g} = c$

so läßt sich der maximal sinnvolle Anteil X durch Gleichung (VII) ausdrücken:

$$X = (c^2 + bc - ((c^2 + bc)^2 - 4bc^2 * (c - a))^{0,5}) / (2c^2) \qquad (VII)$$

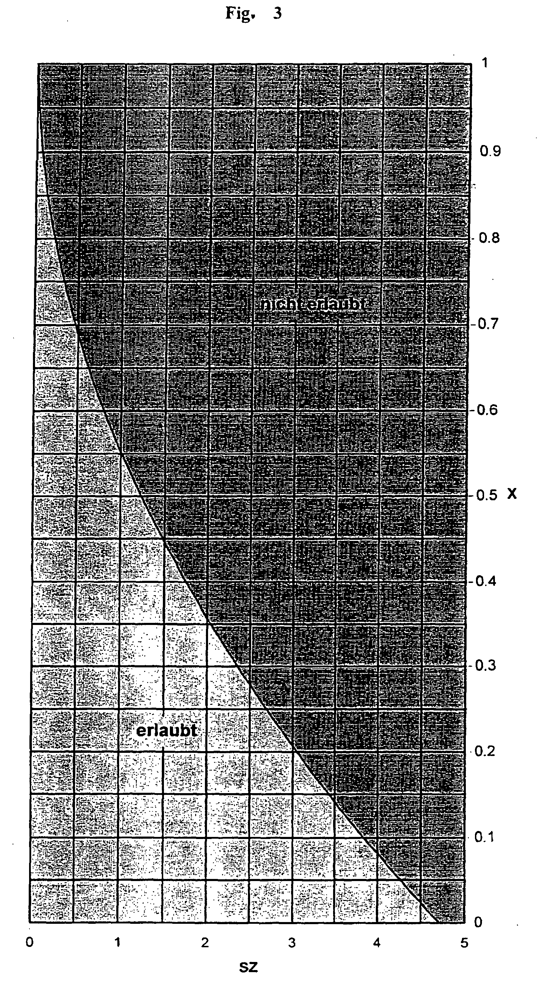

Natürlich ist auch die Wiederverwendung geringerer Anteile der basischen Glycerin-Phase für die Umesterung möglich. Dieser Sachverhalt ist graphisch in Fig. 3 für eine mittlere Molmasse des Öls von 880 g/mol dargestellt: Anteile X, die im hell schraffierten Bereich von Fig. 3 liegen, gewährleisten eine ausreichende Katalysatormenge im verbleibenden Anteil (1-X) für die Neutralisation der freien Fettsäuren.

Nach der vorstehend beschriebenen Umesterungsstufe kann das Reaktionsgemisch in geeigneter Weise aufgearbeitet werden, wobei sich die Phasentrennung als besonders vorteilhaft erwiesen hat.

Hierzu wird das in der Umesterungsstufe erhaltene Gemisch in ein Gefäß geleitet, das vorteilhafterweise um ein vielfaches größer ist als der zur Vorbehandlung und Umesterung verwendete Reaktor. Der Vorteil dieser Dimensionierung ist darin zu sehen, daß dieser Absetzbehälter als Reservoir genutzt werden kann, wodurch einerseits die Absetzzeiten länger als die für die Vorbehandlung und Umesterung benötigte Zeit sein können und andererseits eine kontinuierliche Produktentnahme möglich wird.

Dieser Behälter soll weiterhin so ausgelegt sein, daß die entstehende Restphase, die unter anderem die mehrwertigen Alkohole enthält, und die an Fettsäureestern einwertiger Alkohole reiche Phase durch zwei an verschiedenen Stellen angebrachte Auslässe abgenommen werden können.

Als günstig hat sich erwiesen, das Reaktionsgemisch etwa in Höhe der flüssig-flüssig-Phasengrenze zuzuführen, was durch einen entsprechend angebrachten oder ausgebildeten Einlaß erreicht werden kann und die esterreiche Phase möglichst weit von der Phasengrenze entfernt abzunehmen, d.h. der Auslaß für die esterreiche Phase sollte möglichst nah an der Füllstandsgrenze des zur Phasentrennung verwendeten Behälters angebracht sein.

Diese Optimierung findet natürlich ihre Grenze darin, daß ein zu weit oben angebrachter Auslaß nur kleine Entnahmemengen gestattet, wohingegen ein weiter unten angebrachter Auslaß zwar größere Entnahmemengen gestattet, aber auch ein weniger reines Produkt bedingt. Der hier notwendige Kompromiß kann für eine gegebene Anlage von einem Fachmann ohne weiteres bestimmt werden. Üblicherweise wird der Auslaß im oberen Drittel des Behältnisses liegen.

Aus dem zur Phasentrennung verwendeten Behälter kann oberhalb der flüssig-flüssig-Phasengrenze eine an Fettsäureestern einwertiger Alkylalkohol reiche Phase abgenommen werden und anschließend nach herkömmlichen Verfahren aufgearbeitet werden oder aber einer zweiten Umesterung unterworfen werden.

Eine zweite Umesterung bietet sich immer dann an, wenn eine möglichst vollständige Überführung der im Ausgangsprodukt enthaltenen Fettsäurereste in Fettsäureester einwertiger Alkylalkohole gewünscht wird. Hierzu wird die abgetrennte Phase in einen zweiten Reaktor, der vorteilhaft als Rührkessel gestaltet ist, überführt und mit Alkylalkohol und Base unter den gleichen Reaktionsbedingungen wie in der ersten Umesterungsstufe umgesetzt.

An dieser Stelle muß Alkylalkohol in einer Menge von 5-80%, bevorzugt von 10-20% der oben genannten Gesamtmenge zugegeben werden, da der in den vorherigen Schritten eingesetzte Alkylalkohol bei der Phasentrennung im wesentlichen in die Glycerinphase wandert.

Die benötigte Alkoholmenge kann entweder direkt zugegeben werden oder aber zunächst mit der Base vermischt und dann in den Reaktor geleitet werden. Auch hier sollte vorteilhaft der gleiche Alkylalkohol bzw. die gleiche Alkylalkoholmischung wie in den vorangehenden Stufen eingesetzt werden.

Als Base kommen hier die gleichen Verbindungen wie in der ersten Umesterungsstufe in Betracht, wobei hinsichtlich der Handhabbarkeit wiederum 25-50 Gew.-% alkoholische Lösungen bevorzugt sind.

Die Reaktionszeit in dieser Stufe beträgt 15-45 min, und bevorzugt 25-35 min.

Auch das nach der zweiten Umesterung erhaltene Reaktionsgemisch kann auf eine der bekannten Arten aufgearbeitet werden, wobei sich wiederum eine auf die oben beschriebene Art durchgeführte Phasentrennung als vorteilhaft erwiesen hat. Besonders vorteilhaft ist es, das Reaktionsgemisch vor der letzten Phasentrennung mit mindestens 5 Gew.% Wasser, bezogen auf das Gewicht der in diesem Schritt eingesetzten fettsäurehaltigen Mischung, zu waschen.

Weiterhin können nach der letzten Phasentrennung niedrig siedende Bestandteile aus der an Fettsäureestern einwertiger Alkylalkohole reichen Fraktion ausgetrieben werden. Hierzu wird vorteilhafterweise ein Verdampfer eingesetzt, bei dem die zuletzt abgetrennte Fraktion über eine erste 90 bis 120 °C heiße Oberfläche geleitet wird und die niedrig siedenden Bestandteile auf einer zweiten Oberfläche niedergeschlagen werden.

Im folgenden sei das erfindungsgemäße Verfahren anhand von Beispielen und Vergleichsbeispielen näher erläutert.

Vergleichsbeispiel 1:

500 g kaltgepreßtes Rapsöl mit einem Gehalt an freien Fettsäuren (FFA-Gehalt) von 0,3% werden bei 293 K in einem Becherglas unter Rühren mit 52.5 g Methanol (99,7%) und 25 g einer 30 Gew%igen Lösung von KOH (85%ig) in Methanol versetzt. Nach etwa 25 Minuten wird das Rührwerk abgestellt. Die Mischung wird in einen Scheidetrichter überführt, sodaß nach etwa 3 Stunden die beiden flüssigen Phasen in zwei Behältern aufgefangen werden können. Der Biodiesel wird durch Zugabe von 2 g einer 30%igen Lösung von KOH in Methanol vollständig umgeestert, und abschließend gewaschen und getrocknet. Die Ausbeute beträgt 495 g (99%) Rapsmethylester und 82,5 g basische Glycerin-Phase folgender Zusammensetzung: ca. 65% Glycerin, ca. 18% Methanol, ca. 17% freies KOH und Kaliseifen.

Vergleichsbeispiele 2-5:

Die weiteren Vergleichsbeispiele wurden entsprechend der Vorschrift für Vergleichsbeispiel 1 durchgeführt. Die relevanten Daten aller Vergleichsbeispiele sind in Tabelle 1 zusammengefaßt.

## Tabelle 1

| Vergleichsbeispiel | 1 | 2 | 3 | 4 | 5 |
|---|---|---|---|---|---|
| eingesetzte Menge [g] | 500 | 500 | 500 | 500 000 | 500 |
| Art des Fetts | Rapsöl kaltgepr. | Rapsöl kaltgepr. | Pflanzenöl übersäuert | Friteusenfett | Tierkörperfett |
| FFA-Gehalt [%] | 0.3 | 1 | 3 | 5 | 9 |
| Wassergehalt [%] | - | - | - | 1.9 | - |
| Temperatur [K] | 293 | 293 | 293 | 313 | 313 |
| zugesetzte Menge an Methanol (99.7%) [g] | 52.5 | 52.5 | 46.5 | 41 000 | 31.5 |
| zuges. Menge an KOH (25.5%) in Methanol [g] | 25 | 25 | 33.5 | 41 500 | 55 |
| Dauer der Phasentrennung [h] | 3 | 3 | 3 | 3 | 3 |
| 2. Umesterung: KOH (30%) in Methanol [g] | 2 | 2 | 2 | 2 000 | 2 |
| Ausbeute Fettsäuremethylester [g] | 495 | 480 | 440 | 400 000 | 300 |
| [%] | 99 | 96 | 88 | 80 | 60 |
| bas. Glycerin-Phase [g] | 82.5 | 97.5 | 140 | 182 500 | 286.5 |
| davon [%] Glycerin | 65 | 56 | 39 | 30 | 19 |
| [%] Methanol | 18 | 15 | 11 | 8 | 5 |
| [%] KOH (frei und als Kaliseife) | 17 | 29 | 50 | 62 | 76 |

EP 1 183 225 B1

Tabelle 2

| Beispiel | 1 | 2 | 3 | 4 | 5 |
|---|---|---|---|---|---|
| eingesetzte Menge [g] | 500 | 500 000 | 500 | 500 000 | 500 000 |
| Art des Fetts | Rapsöl kaltgepr. | saures Sojaöl | Tierkörperfett | Friteusenfett | Tierkörperfett |
| FFA-Gehalt [%] | 1 | 4.3 | 9 | 5 | 12 |
| Wassergehalt [%] | 0.2 | 0.35 | 0.8 | 1.9 | 1.2 |
| Temperatur [K] | 293 | 293 | 313 | 313 | 313 |
| zuges. Menge an basische Glycerin-Phase [g] | 50 | 50 000 | 50 | 50 000 | 75 000 |
| Dauer Rühren [min] | 10 | 10 | 10 | 10 | 5 |
| Ausbeute [g] | 495 | 478 000 | 450 | 465 000 | 433 000 |
| FFa-Gehalt [%] | < 0.1 | 0.3 | 0.3 | 0.2 | 0.3 |
| Wassergehalt | < 0.1 | < 0.1 | < 0.1 | 0.1 | 0.1 |

Tabelle 3

| Beispiel | 1 | 2 | 3 | 4 | 5 |
|---|---|---|---|---|---|
| eingesetzte Menge [g] | 495 | 478 000 | | 465 000 | 433 000 |
| zugesetzte Menge an Methanol (99.7%) [g] | 59.3 | 52 600 | | 51 500 | 36 400 |
| zuges. Menge an KOH (28.05%) in Methanol [g] | 12 | 17 200 | | 16 800 | 29 000 |
| Dauer Rühren [min] | 25 | 25 | | 25 | 30 |
| Dauer der Phasentrennung [h] | 1 | 2 | | 3 | 3 |
| 2. Umesterung: KOH (33%) in Methanol [g] | 3 | 4 300 | | 4 200 | 7 300 |
| Ausbeute Fettsäuremethylester [g] | 492.5 | 473 000 | | 460 500 | 428 500 |
| [%] | 99.5 | 99 | | 99 | 99 |
| bas. Glycerin-Phase [g] | 73 | 79 100 | | 77 000 | 77 200 |
| davon [%] Glycerin | 68 | 60 | | 60 | 56 |
| [%] Methanol | 20 | 18 | | 18 | 17 |
| [%] KOH (frei) | 7 | 9 | | 9 | 15 |
| [%] KOH (als Kaliseife) | 4 | 7 | | 7 | 7 |
| [%] Wasser | 1 | 6 | | 6 | 5 |
| Gesamtausbeute [%] | 98.5 | 94.6 | | 92.6 | 86.6 |

EP 1 183 225 B1

Beispiel 1 (Schritt a):

500 g kaltgepreßtes Rapsöl mit einem FFA-Gehalt von 1% und einem Wassergehalt von 0,2% werden bei 293 K in einem Becherglas unter Rühren mit 50 g basischer Glycerin-Phase (60% Glycerin, 20% Methanol, Rest Seifen und freies KOH) versetzt, und anschließend 10 Minuten intensiv gerührt. Die Mischung wird in einen Scheidetrichter überführt und schließlich nach 3 Stunden Sedimentation durch einfaches Ablassen voneinander getrennt. Die Ausbeute betrug 495 g mit einem FFA- und Wassergehalt von weniger als 0,1%.

Beispiele 2 - 5 (Schritt a): Die prinzipielle Vorgehensweise entspricht der von Beispiel 1. Alle relevanten Daten sind in Tabelle 2 zusammengefaßt.

Beispiel 1 (Schritt b):

Die 495 g kaltgepreßtes Rapsöl aus Beispiel 1 (Schritt a) werden bei 293 K in einem Becherglas unter Rühren mit 59.3 g Methanol (99.7%) und 12 g einer 33 Gew%igen Lösung von KOH (85%ig) in Methanol versetzt. Nach etwa 25 Minuten wird das Rührwerk abgestellt und die Mischung in einen Scheidetrichter überführt. Nach etwa 1 Stunde werden die beiden flüssigen Phasen in zwei Behältern aufgefangen. Der Biodiesel wird durch Zugabe von 3 g einer 33%igen Lösung von KOH in Methanol vollständig umgeestert, die entstandene Glycerinphase abgetrennt und mit der ersten vereinigt, und der Biodiesel abschließend gewaschen und getrocknet. Die Ausbeute beträgt 492,5 g (99,5%) Rapsmethylester und 73 g basischer Glycerin-Phase folgender Zusammensetzung:
ca. 68% Glycerin, ca. 20% Methanol, ca. 4% Kaliseifen, ca. 7% freies KOH und ca. 1% Wasser. Die auf den Rohstoff vor der Extraktion bezogene Gesamtausbeute beträgt 98,5%.

Beispiele 2-5 (Schritt b):

Es wurde das Protokoll von Beispiel 1 (Schritt b) angewendet. Die eingesetzten Mengen, Reaktionsbedingungen und Ausbeuten sind in Tabelle 3 zusammengefaßt.

Der Vergleich der erzielten Ausbeuten der Vergleichsbeispiele in Tabelle 1 mit den Gesamtausbeuten nach Tabelle 3 zeigt, daß das konventionelle Verfahren lediglich bei sehr niedrigem Gehalt an freien Fettsäuren mit der vorliegenden Erfindung konkurrieren kann (Beispiel 1 und Vergleichsbeispiel 1).

Je höher der Gehalt an freien Fettsäuren im eingesetzten Ausgangsmaterial ist, desto größer wird der Vorteil der vorliegenden Erfindung.

Bei einem identischen Gehalt an freien Fettsäuren von 5% in Beispiel 4 und in Vergleichsbeispiel 4 ist die Gesamtausbeute nach dem Verfahren der vorliegenden Erfindung mit 92.6% deutlich höher als nach der konventionellen Methode (80%).

Ebenso ist die Gesamtausbeute mit 86.6% für die Umesterung von Tierkörperfett nach dem Verfahren der vorliegenden Erfindung (Beispiel 5) wesentlich besser als nach konventionellen Methoden (60%; Vergleichsbeispiel 5), und das sogar bei höherem Anteil an freien Fettsäuren (Beispiel 5: 12% FFA; Vergleichsbeispiel 5: 9% FFA).

**Patentansprüche**

1. Verfahren zur Herstellung von Fettsäureestern einwertiger Alkylalkohole durch basenkatalysierte Umesterung von Triglyceriden aus natürlichen oder synthetischen Ölen und/oder Fetten, die als störende Begleitstoffe freie Fettsäuren enthalten,
   wobei man zunächst die Öle und/oder Fette mit einer nicht-mischbaren basischen Glycerin-Phase behandelt, so daß die freien Fettsäuren neutralisiert werden und in die Glycerin-Phase übergehen, und dann
   die Triglyceride nach Abtrennung von der Glycerin-Phase mit einwertigen Alkoholen unter Verwendung einer Base als Katalysator in einem Rührkessel zu den Fettsäureestern umestert,
   **dadurch gekennzeichnet, daß**
   man die bei der Umesterung der Triglyceride anfallende basische Glycerin-Phase nach Abtrennung der Fettsäureester für die Behandlung der Öle und/oder Fette zur Entfernung der freien Fettsäuren verwendet, wobei die Mindestmenge an eingesetztem Katalysator bezogen auf 1000 g des zu behandelnden Öls in Abhängigkeit von der Säurezahl und der mittleren Molmasse des Öls gemäß Gleichungen (I) - (III) berechnet wird:

   bei einer Säurezahl, die Ungleichung (I) erfüllt

$$SZ < (0{,}084 \text{ mol}/1000 \text{ g Öl}) * M(KOH) * Y \qquad\qquad (I)$$

gemäß Gleichung (II)

$$\text{Mindestmenge Kat/1000 g Öl} = 0{,}088 \text{ mol/1000 g Öl} * Y \qquad (II)$$

andernfalls gemäß Gleichung (III)

$$\text{Mindestmenge Kat/1000 g Öl} =$$

$$(SZ / M(KOH)) * (0{,}088 / 0{,}084) \qquad (III)$$

mit
Y = (880 g/mol) / (mittlere Molmasse des eingesetzten Öls) und
SZ = Säurezahl des eingesetzten Öls [(g KOH)/(1000 g Öl)].

2. Verfahren nach Anspruch 1, wobei den nach der Vorbehandlung mit einer nicht-mischbaren basischen Glycerin-Phase erhaltenen Triglyceriden zur Umesterung Base und Alkylalkohol zugegeben wird und nach der Umesterung eine an Fettsäureestern einwertiger Alkylalkoholen reiche Fraktion abgetrennt wird, wobei die abgetrennte erste, an Fettsäureestern einwertiger Alkylalkohole reiche Fraktion, zur weiteren Umesterung mit Base und Alkylalkohol versetzt wird und nach der Umesterung eine zweite an Fettsäureestern einwertiger Alkohole reiche Fraktion abgetrennt wird.

3. Verfahren nach Anspruch 1 oder 2, wobei als Base Metallhydroxide und/oder -alkoholate oder deren alkylalkoholische Lösungen verwendet werden.

4. Verfahren nach einem der vorstehenden Ansprüche, wobei die Abtrennung der an Fettsäureestern einwertiger Alkylalkohole reichen Fraktionen durch Phasentrennung erfolgt.

5. Verfahren nach einem der vorstehenden Ansprüche, wobei vor der jeweils letzten Phasentrennung das Reaktionsgemisch mit 5 oder mehr Gew.% Wasser, bezogen auf das Gewicht der in diesem Schritt eingesetzten fettsäureesterhaltigen Mischung, gewaschen wird.

6. Verfahren nach Anspruch 5, wobei nach der jeweils letzten Phasentrennung aus der an Fettsäureestern einwertiger Alkylalkohole reichen Fraktion niedrig siedende Bestandteile ausgetrieben werden.

7. Verfahren nach einem der Ansprüche 4 bis 6, wobei zur Abtrennung der an Fettsäureestern einwertiger Alkylalkohole reichen Fraktionen ein Behältnis verwendet wird, in das das jeweilige Ausgangsgemisch an oder unterhalb der flüssig-flüssig-Phasengrenze zugeführt wird und die abzutrennende Fraktion im oberen Drittel des Behältnisses abgenommen wird.

8. Verfahren nach Anspruch 6 oder 7, wobei zur Austreibung der niedrigsiedenden Bestandteile ein Verdampfer eingesetzt wird, bei dem die zuletzt abgetrennte Fraktion über eine erste 90 bis 120°C heiße Oberfläche geleitet wird und die niedrigsiedenden Bestandteile auf einer zweiten Oberfläche niedergeschlagen werden.

9. Verfahren nach wenigstens einem der vorstehenden Ansprüche, wobei als Ausgangsmaterial pflanzliche Öle verwendet werden.

10. Verfahren nach wenigstens einem der vorstehenden Ansprüche, wobei Alkylalkohole mit 1 bis 10 Kohlenstoffatomen verwendet werden.

11. Verfahren nach wenigstens einem der vorstehenden Ansprüche, wobei die Vorbehandlung mit der basischen Glycerin-Phase in einem Rührkessel über eine Dauer von 1 bis 60 min. durchgeführt wird.

12. Verfahren nach Anspruch 11, wobei die Dauer der Vorbehandlung 5 bis 15 min. beträgt.

13. Verfahren nach wenigstens einem der vorstehenden Ansprüche, wobei 5 bis 15% der bei der Umesterung entstandenen basischen Glycerin-Phase in dem Vorbehandlungsschritt eingesetzt werden.

**14.** Verfahren nach wenigstens einem der Ansprüche 1 bis 12, wobei zusätzlich zu der bei der Umesterung entstandenen basischen Glycerin-Phase weiteres Glycerin und/oder Base zur Entfernung der freien Fettsäuren eingesetzt werden.

**15.** Verfahren nach Anspruch 14, wobei zusätzlich technisches Glycerin oder Pharmaglycerin, in dem KOH, NaOH, oder Natriumethylat gelöst sind, eingesetzt werden.

**16.** Verfahren nach wenigstens einem der vorstehenden Ansprüche, wobei die eingesetzte Gesamtmenge an basischem Umesterungskatalysator das 1 bis 4-fache der Mindestmenge an Katalysator gemäß Anspruch 1 beträgt.

**17.** Verfahren nach einem oder mehreren der vorstehenden Ansprüche, wobei die Gesamtmenge an eingesetztem Alkohol 1,2 bis 1,4 mol pro mol gebundener Fettsäureanteile beträgt.

**18.** Verfahren nach wenigstens einem der vorstehenden Ansprüche, **dadurch gekennzeichnet, daß** ein Anteil X der bei der Umesterung der Triglyceride anfallenden basischen Glycerin-Phase nach Abtrennung der Fettsäureester für die Umesterungsreaktion wiederverwendet wird, wobei die Säurezahl Ungleichung (IV) erfüllt

$$\text{SZ} < (0{,}084 \text{ mol}/1000 \text{ g Öl}) * M(KOH) * Y \qquad (IV)$$

und die einzusetzende Mindestmenge an Katalysator (bezogen auf 1000 g des zu behandelnden Öls) sowie der Anteil X der für die Umesterung wiedereinzusetzenden basischen Glycerin-Phase so gewählt werden, daß gleichzeitig Gleichungen (V) und (VI) erfüllt werden:

$$\text{Mindestmenge Kat}/1000 \text{ g Öl} =$$

$$(0{,}088 \text{ mol}/1000 \text{ g Öl} - (X * 0{,}084 \text{ mol}/1000 \text{ g Öl})) * Y \qquad (V)$$

$$\text{Mindestmenge Kat}/1000 \text{ g Öl} =$$

$$(\text{SZ} / M(KOH)) * (0{,}088 / (0{,}084 * (1 - X))) \qquad (VI)$$

mit

Y = (880 g/mol) / (mittlere Molmasse des eingesetzten Öls)
SZ = Säurezahl des eingesetzten Öls [(g KOH)/(1000 g Öl)].
X = Anteil der basischen Glycerin-Phase, der für die Umesterung wiederverwendet wird.

**19.** Verfahren nach wenigstens einem der vorstehenden Ansprüche, **dadurch gekennzeichnet, daß** die mit der basischen Glycerin-Phase abgetrennten freien Fettsäuren bzw. deren Salze mit Alkylalkohol nachverestert werden.

**20.** Verfahren zur Herstellung von Kraftstoff für Dieselmotoren, umfassend ein Verfahren zur Herstellung von Fettsäureestern einwertiger Alkylalkohole gemäß einem der Ansprüche 1-19.

**Claims**

**1.** Process for producing fatty acid esters of monohydric alkyl alcohols by base-catalysed transesterification of triglycerides from natural or synthetic oils and/or fats, which contain free fatty acids as interfering accompanying materials, wherein first of all the oils and/or fats are treated with a non-miscible basic glycerine phase, so that the free fatty acids are neutralised and are converted into the glycerine phase, and then the triglycerides, after separating off from the glycerine phase using monohydric alcohols, are transesterified to form the fatty acid esters using a base as catalyst in a stirred vessel, **characterised in that** the basic glycerine phase accumulating during transesterification of the triglycerides, after separating off the fatty acid esters, is used for the treatment of the oils and/or fats for removing the free fatty acids, wherein the minimum quantity of catalyst used, based on 1,000 g of the oil to be treated, is calculated as a function of the acid number and the average molar weight of the oil according to equations (I) - (III):

for an acid number which fulfils inequation (I)

$$AN < (0.084 \text{ mole}/1{,}000 \text{ g of oil}) * M(KOH) * Y \qquad (I)$$

according to equation (II)

$$\text{minimum quantity of catalyst}/1{,}000 \text{ g of oil} =$$

$$0.088 \text{ mole}/1{,}000 \text{ g of oil} * Y \qquad (II)$$

otherwise according to equation (III)

$$\text{minimum quantity of catalyst}/1{,}000 \text{ g of oil} =$$

$$(AN/M(KOH))*(0.088/0.084) \qquad (III)$$

where

Y = (880 g/mole)/(average molar weight of the oil used)
and
AN = acid number of the oil used [(g KOH)/(1,000 g of oil)].

2. Process according to claim 1, wherein base and alkyl alcohol is added to the triglycerides obtained after pretreatment with a non-miscible basic glycerine phase for transesterification and after transesterification, a fraction rich in fatty acid esters of monohydric alkyl alcohols is separated off, wherein the separated first fraction rich in fatty acid esters of monohydric alkyl alcohols is treated with base and alkyl alcohol for further transesterification and after transesterification, a second fraction rich in fatty acid esters of monohydric alcohols is separated off.

3. Process according to claim 1 or 2, wherein metal hydroxides and/or metal alcoholates or their alkyl alcoholic solutions are used as base.

4. Process according to one of the above claims, wherein separation of the fractions rich in fatty acid esters of monohydric alkyl alcohols is effected by phase separation.

5. Process according to one of the above claims, wherein before the particular last phase separation, the reaction mixture is washed with 5 or more wt.% of water, based on the weight of the fatty acid ester-containing mixture used in this step.

6. Process according to claim 5, wherein after the particular last phase separation from the fraction rich in fatty acid esters of monohydric alkyl alcohols, low-boiling constituents are expelled.

7. Process according to one of claims 4 to 6, wherein for separating off the fractions rich in fatty acid esters of monohydric alkyl alcohols, a container is used, in which the particular starting mixture is supplied at or below the liquid-liquid phase boundary and the fraction to be separated off is removed in the upper third of the container.

8. Process according to claim 6 or 7, wherein to expel the low-boiling constituents, an evaporator is used, in which the fraction separated off last of all is passed over a first surface heated at 90 to 120°C and the low-boiling constituents are deposited on a second surface.

9. Process according to at least one of the above claims, wherein vegetable oils are used as starting material.

10. Process according to at least one of the above claims, wherein alkyl alcohols having 1 to 10 carbon atoms are used.

**11.** Process according to at least one of the above claims, wherein pre-treatment with the basic glycerine phase is carried out in a stirred vessel over a period of 1 to 60 minutes.

**12.** Process according to claim 11, wherein the period of pre-treatment is 5 to 15 minutes.

**13.** Process according to at least one of the above claims, wherein 5 to 15 % of the basic glycerine phase produced during transesterification is used in the pre-treatment step.

**14.** Process according to at least one of claims 1 to 12, wherein in addition to the basic glycerine phase produced during transesterification, further glycerine and/or base are used to remove the free fatty acids.

**15.** Process according to claim 14, wherein additionally technical glycerine or pharmaglycerine, in which KOH, NaOH, or sodium ethylate are dissolved, are used.

**16.** Process according to at least one of the above claims, wherein the total quantity of basic transesterification catalyst used is I to 4 times the minimum quantity of catalyst according to claim 1.

**17.** Process according to one or more of the above claims, wherein the total quantity of alcohol used is 1.2 to 1.4 moles per mole of bound fatty acid portions.

**18.** Process according to at least one of the above claims, **characterised in that** a portion X of the basic glycerine phase accumulating during transesterification of the triglycerides is re-used after separating off the fatty acid esters for the transesterification reaction, wherein the acid number fulfils inequation (IV)

$$AN < (0.084 \text{ mote}/1{,}000 \text{ g of oil}) * M(KOH) * Y \tag{IV}$$

and the minimum quantity of catalyst to be used (based on 1,000 g of the oil to be treated) as well as the portion X of the basic glycerine phase to be re-used for transesterification are selected so that at the same time equations (V) and (VI) are fulfilled:

$$\text{minimum quantity of catalyst}/1{,}000 \text{ g of oil} =$$

$$(0.088 \text{ mole}/1{,}000 \text{ g of oil} - (X * 0.084 \text{ mole}/1{,}000 \text{ g of oil})) *Y \tag{V}$$

$$\text{minimum quantity of catalyst}/1{,}000 \text{ g of oil} =$$

$$(AN/M(KOH)) * (0.088/0.084 * (1 - X)) \tag{VI}$$

where

$Y = (880 \text{ g/mole})/(\text{average molar weight of the oil used})$
$AN$ = acid number of the oil used [(g KOH)/(1,000 g of oil)]
$X$ = portion of basic glycerine phase, which is re-used for transesterification.

**19.** Process according to at least one of the above claims, **characterised in that** the free fatty acids or their salts separated off with the basic glycerine phase are post-esterified with alkyl alcohol.

**20.** Process for producing fuel for diesel engines, comprising a process for producing fatty acid esters of monohydric alkyl alcohols according to one of claims 1-19.

**Revendications**

**1.** Procédé de production d'esters d'acides gras d'alcools alkyliques monovalents par transestérification sous catalysation basique de triglycérides issus d'huiles et/ou de graisses naturelles ou synthétiques, contenant des acides gras libres, à titre de substances d'accompagnement perturbatrices,

où, d'abord, on traite les huiles et/ou les graisses avec une phase glycérine basique non miscible, de manière que les acides gras libres soient neutralisés et se transforment en phase glycérine et, ensuite,

les triglycérides, après séparation de la phase glycérine, étant transestérifiés en les esters d'acides gras, avec des alcools monovalents avec utilisation d'une base comme catalyseur, dans un récipient d'agitation,

**caractérisé en ce que** l'on utilise lors de la transestérification des triglycérides la phase glycérine basique apparaissant, après séparation des esters d'acides gras, pour le traitement des huiles et/ou des graisses, dans le but d'éliminer les acides gras libres, la quantité minimale de catalyseur utilisée, rapportée à 1000 g de l'huile à traiter, étant calculée, en fonction de l'indice d'acidité et de la masse molaire moyenne de l'huile, selon les équations (I) à (III) :

pour un indice d'acidité SZ, qui satisfait l'inéquation (I):

$$SZ < (0,084 \text{ mole/1000 g d'huile}) * M(KOH) * Y \qquad (I),$$

selon l'équation (II)

$$\text{Quantité minimale de catalyseur/1000 g}$$

$$\text{d'huile} = 0,088 \text{ mole/1000 g d'huile} * Y \qquad (II)$$

Et, autrement, selon l'équation (III)

$$\text{Quantité minimale de catalyseur/1000 g d'huile}$$

$$= (SZ / M (KOH)) * (0.088 / 0.084), \qquad (III)$$

avec

Y = (880 g/mol) / (masse molaire moyenne de l'huile utilisée) et
SZ = indice d'acidité de l'huile utilisée [(g KOH)/(1000 g d'huile)]

2. Procédé selon la revendication 1, dans lequel on ajoute aux triglycérides obtenus après prétraitement avec une phase glycérine basique non miscibles, dans le but d'effectuer la transestérification, une base et un alcool alkylique et, après la transestérification, on sépare une fraction, riche en esters d'acides gras, d'alcools alkyliques monovalents, la première fraction séparée, riche en esters d'acides gras d'alcools alkyliques monovalents, étant mélangée à une base et à de l'alcool alkylique dans le but d'obtenir une transestérification supplémentaire et, après la transestérification, une deuxième fraction, riche en esters d'acides gras d'alcools monovalents, étant séparée.

3. Procédé selon la revendication 1 ou 2, dans lequel on utilise comme base des hydroxydes et/ou des alcoolats métalliques ou bien leurs solutions dans un alcool alkylique.

4. Procédé selon l'une des revendications précédentes, la séparation des fractions, riches en esters d'acides gras, d'alcools alkyliques monovalents se faisant par séparation des phases.

5. Procédé selon l'une des revendications précédentes, où, avant la dernière séparation des phases chaque fois effectuée, le mélange de réaction est lavé avec 5 % en poids d'eau ou plus, en se référant au poids du mélange, contenant un ester d'acide gras, utilisé dans cette étape.

6. Procédé selon la revendication 5, où après la dernière séparation de phase chaque fois effectuée, on chasse les composants à faible point d'ébullition de la fraction riche en esters d'acides gras d'alcools alkyliques monovalents.

7. Procédé selon l'une des revendications 4 à 6, où, pour effectuer la séparation des fractions riches en esters d'acides gras d'alcools alkyliques monovalents, on utilise un récipient dans lequel le mélange initial respectif est amené à ou au-dessous de la limite de phase liquide-liquide et la fraction à séparer est prélevée dans le tiers supérieur du récipient.

**8.** Procédé selon la revendication 6 ou 7, où, pour chasser les composants à faible point d'ébullition, on utilise un évaporateur pour lequel la dernière fraction séparée est guidée sur une première surface chaude, d'une température de 90 à 120°C. et les composants à faibles point d'ébullition étant déposés sur une deuxième surface.

**9.** Procédé selon au moins l'une des revendications précédentes, des huiles végétales étant utilisées comme matériau de départ.

**10.** Procédé selon au moins l'une des revendications précédentes, des alcools alkyliques, comportant de 1 à 10 atomes de carbone, étant utilisés.

**11.** Procédé selon au moins l'une des revendications précédentes, où le prétraitement est effectué avec la phase glycérine basique, dans un récipient d'agitation, sur une durée de 1 à 60 min.

**12.** Procédé selon la revendication 11, la durée du prétraitement étant de 5 à 15 min.

**13.** Procédé selon au moins l'une des revendications précédentes, où de 5 à 15 % de la phase glycérine basique produite lors de la transestérification sont utilisés dans l'étape de prétraitement.

**14.** Procédé selon au moins l'une des revendications 1 à 12, où, en plus de la phase glycérine basique produite lors de la transestérification, d'autres glycérines et/ou bases sont utilisées pour l'élimination des acides gras libres.

**15.** Procédé selon la revendication 14, où, en plus, est utilisée de la glycérine technique ou de la pharmaglycérine, dans laquelle sont dissous KOH, NaOH, ou de l'éthylate de sodium.

**16.** Procédé selon au moins l'une des revendications précédentes, la quantité globale utilisée de catalyseur basique de transestérification étant de 1 à 4 fois la quantité minimale de catalyseur selon la revendication 1.

**17.** Procédé selon l'une ou plusieurs des revendications précédentes, la quantité globale d'alcool utilisé étant de 1,2 à 1,4 mole par mole de fraction d'acide gras liée.

**18.** Procédé selon au moins l'une des revendications précédentes, **caractérisé en ce qu'**une proportion X de la phase glycérine basique, apparaissant lors de la transestérification des triglycérides, est réutilisée pour la réaction de transestérification, après séparation des esters d'acides gras, l'indice d'acidité satisfaisant à l'inégalité (IV) :

$$SZ < (0{,}084 \text{ mole/1000 g d'huile}) * M(KOH) * Y \qquad (IV)$$

et la quantité minimale à utiliser de catalyseur (en se référant à 1000 g de l'huile à traiter), ainsi que la fraction X de la phase glycérine basique à réutiliser pour la transestérification, étant choisies de manière que l'on satisfasse simultanément aux équations (V) et (VI) :

$$\text{Quantité minimale de catalyseur/1000 g}$$

$$\text{d'huile} = (0{,}088 \text{ mole/1000 g d'huile}$$

$$- (X * 0{,}084 \text{ mole/1000 g d'huile})) * Y \qquad (V)$$

$$\text{Quantité minimale de catalyseur/1000 g d'huile} =$$

$$(SZ / M(KOH) * (0{,}088 / (0{,}084 * (1 - X))) \qquad (VI)$$

avec

$Y = (880 \text{ g/mole}) / (\text{masse molaire moyenne de l'huile utilisée})$

SZ = indice d'acidité de l'huile utilisée [(g KOH)/(1000 g d'huile)]
X = proportion de la phase glycérine basique réutilisée pour la transestérification.

19. Procédé selon au moins l'une des revendications précédentes **caractérisé en ce que** les acides gras libres séparés avec la phase glycérine basique, où leurs sels sont soumis à une post-estérification avec un alcool alkylique.

20. Procédé pour la fabrication de carburant pour des moteurs diesel comprenant un procédé de fabrication d'esters d'acides gras d'alcools alkyliques monovalents selon l'une des revendications 1 à 19.

Fig. 1

Neutralisation und Extraktion

Phasentrennung

Umesterung

einwertiger Alkylalkohol + Base

Phasentrennung

Produkt

**Rohmaterial:** Triglycerid + freie Fettsäuren

basische Glycerin-Phase

Triglycerid *unpolar* / *polar* Glycerin + neutralisierte Fettsäuren

Triglycerid + einwertiger Alkylalkohol + Base → Glycerin + Fettsäureester

Fettsäureester *unpolar* / *polar* Glycerin + Base

Glycerinaufbereitung

basische Glycerin-Phase

Fig. 2

*Neutralisation und Extraktion* · *Phasentrennung* · *Umesterung* · *Phasentrennung*

einwertiger Alkylalkohol + Base

Rohmaterial:
Triglycerid + freie Fettsäuren

basische Glycerin-Phase

Triglycerid

unpolar / polar

Glycerin + neutralisierte Fettsäuren

Triglycerid + einwertiger Alkylalkohol | Base

Glycerin + Fettsäureester

Fettsäureester

unpolar / polar

Glycerin + Base

Produkt

Glycerinaufbereitung

Anteil: X

Anteil: 1-X

basische Glycerin-Phase

EP 1 183 225 B1

## Fig. 3